# EUROPÄISCHE PATENTSCHRIFT

(11) **EP 0 655 446 B1**
(45) Veröffentlichungstag und Bekanntmachung des Hinweises auf die Patenterteilung: **07.05.1997**
(21) Anmeldenummer: 94114238.2
(22) Anmeldetag: 09.09.1994
(51) Int. Cl.: C07D 275/04

(54) **Verfahren zur Herstellung von 1,2-Benzisothiazolen**
Process for the production of 1,2-benzisothiazoles
Procédé pour la préparation de 1,2-benzoisothiazoles

(30) Priorität: 13.09.1993 CH 2736/93; 06.07.1994 CH 2159/94
(43) Veröffentlichungstag der Anmeldung: 31.05.1995
(73) Patentinhaber: Novartis AG, 4058 Basel (CH)
(72) Erfinder: Känel, Hans-Ruedi, Dr., CH-4416 Bubendorf (CH); Wegmann, Arthur, CH-4336 Kaisten (CH); Neff, Denis, Dr., CH-1870 Monthey (CH)
(74) Vertreter: Zumstein, Fritz, Dr.

(56) Entgegenhaltungen:
- EP-A- 0 530 136
- J. CHEM. SOC., PERKIN TRANS. I, 1973 Seiten 356 - 359 K. CLARKE ET AL. '1,2-Benzisothiazoles. Part IV. Preparation of the 3-Methyl Derivative from o-Mercaptoacetophenone Oxime: a Re-examination'
- ANN. CHIM. (ROME), Bd.53, Nr.5, 1963 Seiten 577 - 587 A. RICCI, A. MARTANI 'Nuove sintesi del benzoisotiazolo'
- PHOSPHORUS AND SULFUR, Bd.12, 1982 Seiten 357 - 367 A. J. LAWSON 'Thermal Fission of Hydroxylamine Derivatives with Neighbouring-Group Participation by Thioether Functions: Preparation of 1,2-Benzoisothiazoles'
- ADV. HETEROCYCL. CHEM., Bd.38, 1985 Seiten 106 - 133 M. DAVIS 'Recent Advances in the Chemistry of Benzoisothiazoles and Other Polycyclic Isothiazoles'
- CAN. J. CHEM., Bd.66, Nr.6, 1988 Seiten 1405 - 1409 D.M. MCKINNON, K. R. LEE 'Fused heterocycles from o-acylbenzenethiol derivatives'

## Beschreibung

Die Erfindung betrifft ein Verfahren zur Herstellung von 1,2-Benzisothiazolen der Formel I 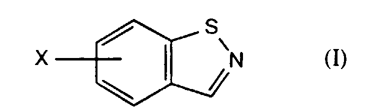 worin
X in einer der 4 möglichen Positionen des Benzolringes steht und Wasserstoff, Halogen, Nitro, C₁-C₄-Alkoxy, Halogen-C₁-C₄-alkoxy, C₂-C₄-Alkenyloxy oder C₂-C₄-Alkinyloxy bedeutet,
dadurch gekennzeichnet, dass eine Verbindung der Formel II 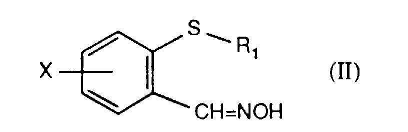 worin X die für Formel I angegebenen Bedeutungen hat und
R₁ Wasserstoff oder einen gegebenenfalls substituierten C₁-C₁₂-Kohlenwasserstoffrest bedeutet, in Gegenwart einer katalytischen Menge einer starken Säure umgesetzt wird.

Verbindungen der Formel I sind als Schädlingsbekämpfungsmittel, insbesondere als Nematizide bekannt (vgl. z.B. EP-A- 454 621 und EP-A-530 136).

Es ist bekannt, dass Benzisothiazole aus 2-(Alkylthio)benzaldehyden durch Reaktion mit Hydroxylamin-O-sulfonsäure und anschliessendem Ringschluss in Lösung hergestellt werden können (vgl. z.B. EP-A-454 621). Bei dieser Synthese wird vorübergehend ein Reaktand ins Molekül eingeführt, von dem der grösste Teil bei der Ringschlussreaktion wieder entfernt wird; die daraus entstehenden Nachteile für eine technische Synthese sind ein unerwünschter Materialumschlag, eine ungenügende Volumenausbeute und eine grosse Abwasserbelastung. Ein weiter Nachteil dieses Verfahrens besteht darin, dass das Reaktionsgemisch im Bereich der Reaktionstemperatur thermisch instabil ist und somit ein Sicherheitsrisiko darstellen kann.

Es ist ferner bekannt, dass Benzisothiazole hergestellt werden können durch Ringschluss von 2-Mercaptobenzaldehydoximen (vgl. z.B. Ann. Chim.(Rome) Vol.53, 1963, Seiten 577-587) oder durch Ringschluss von 2-(Alkylthio)benzaldehydoximen (vgl. z.B. Synthesis 1978, Seiten 58-60; EP-A-530 136). In diesen Verfahren wird als Lösungsmittel eine starke Säure, wie Polyphosphorsäure, oder Phosphorpentoxid in Methansulfonsäure verwendet. Diese Lösungsmittel bzw. Säuren, die in grossem Ueberschuss eingesetzt werden, können wegen ihres hohen Siedepunktes nicht destillativ vom Produkt abgetrennt, sondern müssen durch Verwässerung vom Produkt entfernt werden und belasten so in ausserordentlichem Mass das Abwasser. Als weiterer Nachteil kommt hinzu, dass die Ausbeuten zum Teil schlecht sind.

Die bekannten Verfahren zur Herstellung von Benzisothiazolen der Formel I sind somit aus wirtschaftlichen, ökologischen und sicherheitstechnischen Gründen unbefriedigend.

Es wurde nun überraschenderweise gefunden, dass Benzisothiazole der Formel I in sehr hohen Ausbeuten und guter Reinheit erhalten werden, wenn eine Verbindung der Formel II in einem Lösungsmittel mit katalytischen Mengen einer starken Säure umgesetzt wird.

Das erfindungsgemässe Verfahren hat gegenüber den vorbekannten Verfahren folgende Vorteile:
- Ausbeuten über 90%, z.B. bis 97%;
- weniger Nebenproduktbildung, dadurch leichtere Aufarbeitung;
- verbesserte Volumenausbeuten;
- problemlose Wiedergewinnung des Lösungsmittels;
- geringere Abwasserbelastung (Oekologie);
- keine Gefahr thermischer Zersetzung des Reaktionsgemisches;
- weniger Korrosion des Reaktors.

Die vor- und nachstehend verwendeten Allgemeinbegriffe haben folgende Bedeutung:

Halogen bedeutet z.B. Fluor, Chlor, Brom oder Jod, bevorzugt sind Fluor, Chlor und Brom.

Alkoxy, Alkenyloxy und Alkinyloxy können, je nach Anzahl der Kohlenstoffatome, geradkettig oder verzweigt sein.

Alkoxy ist beispielsweise Methoxy, Ethoxy, Propyloxy, i-Propyloxy, n-Butyloxy, iso-Butyloxy, sek.-Butyloxy und tert.-Butyloxy; vorzugsweise Methoxy und Ethoxy.

Halogenalkoxy kann gleiche oder verschiedene Halogenatome enthalten, z.B. Difluormethoxy, Trifluormethoxy, Chlorfluormethoxy, 2,2,2-Trifluorethoxy, 1,1,2,2-Tetrafluorethoxy, 2-Fluorethoxy, 2-Chlorethoxy und 2,2-Difluorethoxy; vorzugsweise Difluormethoxy.

Alkenyloxy ist z.B. Allyloxy, Methallyloxy oder But-2-en-1-yloxy. Bevorzugt ist Allyloxy.

Alkinyloxy ist z.B. Propargyloxy, But-1-in-1-yloxy, But-2-in-3-yloxy; bevorzugt ist Propargyloxy.

Kohlenwasserstoffreste für R₁können gesättigt oder ungesättigt, verzweigt oder unverzweigt, offenkettig oder cyclisch, aromatisch oder araliphatisch sein. Bevorzugt sind C₁-C₆-Alkyl, C₃-C₆-Cycloalkyl, Phenyl und Benzyl.

Beispiele für offenkettige gesättigte Kohlenwasserstoffreste sind Methyl, Ethyl, n-Propyl, iso-Propyl, n-Butyl, sek.-Butyl, iso-Butyl, tert.-Butyl, sek. Amyl, tert. Amyl, 1-Hexyl oder 3-Hexyl.

Beispiele für offenkettige ungesättigte Kohlenwasserstoffreste sind Allyl, Methallyl, 1-Methylvinyl oder But-2-en-1-yl, Propargyl, But-1-in-1-yl, But-1-in-3-yl.

Cyclische Kohlenwasserstoffreste können aromatisch sein wie z.B. Phenyl und Naphtyl, oder nicht aromatisch wie z.B. Cyclopentenyl, Cyclohexenyl, Cycloheptenyl und Cyclooctadienyl, oder teilaromatisch wie z.B. Tetrahydronaphtyl und Indanyl oder cycloaliphatisch wie Cyclopropyl, Cyclobutyl, Cyclopentyl, Cyclohexyl und Cycloheptyl, oder araliphatisch wie z.B. Benzyl.

R₁ kann beliebig substituiert sein, sofern der Substituent unter den angegebenen Reaktionsbedingungen keinen nachteiligen Effekt auf die Reaktion ausübt. Beispiele für geeignete Substituenten an R₁ sind Phenyl, Halogen und Alkoxy.

Die Reaktion kann mit oder ohne Lösungsmittel durchgeführt werden.

Lösungsmittel sind z.B. aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ether, wie Diethylether, tert.-Butylmethylether, Tetrahydrofuran oder Dioxan; Ketone, wie Aceton oder Methylethylketon; Alkohole, wie Methanol, Ethanol, Propanol, Butanol, Ethylenglycol oder Glycerin; Ester, wie Essigsäureethylester oder Essigsäurebutylester; Amide, wie N,N-Dimethylformamid, N,N-Dimethylacetamid, N-Methylpyrrolidon oder Hexamethylphosphorsäuretriamid; Nitrile, wie Acetonitril; Sulfone, wie Sulfolan; und Harnstoffe, wie Dimethylpropylenharnstoff; ferner Wasser.

Bei Verwendung von unpolaren Lösungsmitteln wie z.B. Benzol, Toluol, Xylol, Chlorbenzol, Brombenzol, Petrolether, Hexan, Cyclohexan, kann es vorteilhaft sein, einen geeigneten Phasentransferkatalysator zuzusetzten. Als Beispiele seien genannt: Tetrabutylammoniumbromid, Dibenzyldimethylammoniummethylsulfat oder Tris[2-(2-methoxyethoxy)ethyl]amin.

In einer bevorzugten Ausführungsform zur Herstellung einer Verbindung der Formel I wird ein polares Lösungsmittel, insbesondere Wasser oder ein C₁-C₆-Alkohol oder ein Gemisch derselben, verwendet. Besonders bevorzugt sind 1-Propanol und 2-Butanol.

Starke Säuren sind z.B. Mineralsäuren wie Halogenwasserstoffsäuren (Fluorwasserstoffsäure, Chlorwasserstoffsäure, Bromwasserstoffsäure oder Jodwasserstoffsäure), Schwefelsäure, Phosphorsäure, Polyphosphorsäure, Salpetersäure; organische Säuren wie Ameisensäure, Essigsäure, Trifluoressigsäure, Trichloressigsäure; Sulfonsäuren wie Benzolsulfonsäure, p-Toluolsulfonsäure, Methansulfonsäure, Hydroxylamin-O-sulfonsäure; Sulfinsäuren wie p-Toluolsulfinsäure.

Bevorzugt herstellbar sind Verbindungen der Formel I, worin
X Halogen oder Nitro bedeuten, besonders bevorzugt solche, worin X in 7-Stellung stehen, ganz besonders bevorzugt ist 7-Chlorbenzisothiazol.

Bevorzugte Verbindungen der Formel II zur Durchführung des erfindungsgemässen Verfahrens sind solche, worin R₁ einen gegebenenfalls substituierten C₁-C₁₂-Kohlenwasserstoffrest bedeutet, bevorzugt C₁-C₆-Alkyl oder Benzyl, ganz besonders solche, worin R₁ tert.Butyl bedeutet.

Als Katalysator wird bevorzugt eine Mineralsäure oder eine Sulfonsäure, besonders bevorzugt p-Toluolsulfonsäure, Methansulfonsäure, Hydroxylamin-O-sulfonsäure oder Schwefelsäure eingesetzt.
Die Säure kann auch an ein festes Trägermaterial gebunden sein, z.B. an ein Ionentauscherharz wie Amberlyst.

Die Katalysatormenge beträgt 1-50 Mol% , vorzugsweise 2-15 Mol% bezogen auf die Verbindung der Formel II.

Die Reaktionstemperatur kann zwischen 30°C und der Rückflusstemperatur des Lösungsmittels variiert werden; vorteilhaft arbeitet man bei der Rückflusstemperatur des Lösungsmittels und destilliert während der Reaktion laufend Lösungsmittel ab.

In der Praxis kann man z.B. wie folgt vorgehen: alle Edukte werden vorgelegt, das Reaktionsgemisch wird zum Siedepunkt erhitzt, während 4-8 Stunden am Rückfluss gehalten, danach das Lösungsmittel, gegebenenfalls unter Vakuum, abdestilliert und anschliessend der Rückstand mit Wasser versetzt. Das Produkt kristallisiert aus und wird abfiltriert.
In einer bevorzugten Ausführungsform wird das Lösungsmittel während der Reaktion, gegebenenfalls unter leichtem Vakuum, laufend abdestilliert. Dadurch kann die Reaktionszeit verkürzt werden.

Das erfindungsgemässe Verfahren kann sowohl batchweise als auch kontinuierlich durchgeführt werden. Bei einer kontinuierlichen Reaktionsführung ist es vorteilhaft, das Reaktionsgemisch mit oder ohne Lösungsmittel über eine festes Trägermaterial zu leiten, an das die Säure als Katalysator gebunden ist.

Die Edukte der Formel II können nach bekannten Methoden gemäss folgendem Reaktionsschema hergestellt werden (vgl. z.B. EP-A- 454 621 und EP-A-530 136):

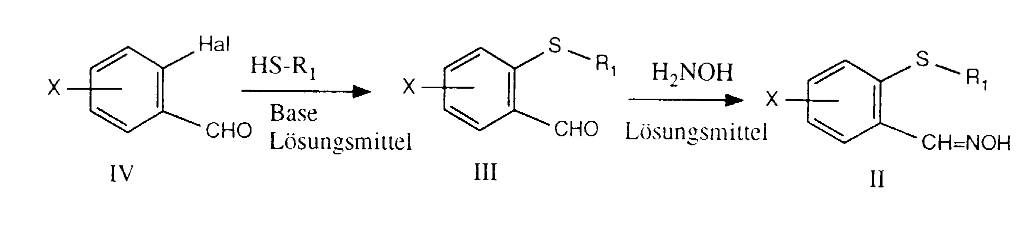

Dabei haben R₁ und X die vorstehend genannten Bedeutungen; Hal bedeutet Halogen. Bevorzugt ist das Verfahren für Verbindungen, worin X in 3-Stellung zur Carbaldehyd-, bzw. Carbaldoxim-Gruppe steht, X und Hal Chlor und R₁ tert.Butyl bedeuten.

Bevorzugte Basen für die Herstellung von Verbindungen der Formel III sind Alkoholate, Carbonate oder Hydrogencarbonate von Alkalimetallen, insbesondere Natriumcarbonat und Kaliumcarbonat; bevorzugte Lösungsmittel sind Alkohole oder aprotische polare Lösungsmittel, besonders bevorzugt sind Dimethylformamid, Dimethylacetamid, Dimethylsulfoxid, N-Methylpyrrolidon und Tetrahydrofuran.

Verbindungen der Formel II können durch Umsetzung einer Verbindung der Formel III mit Hydroxylamin in einem polaren Lösungsmittel wie Wasser oder einem C₁-C₆-Alkohol oder einem Gemisch derselben hergestellt werden; besonders bevorzugt sind Wasser, Methanol, 1-Propanol und 2-Butanol sowie ein Gemisch derselben; ganz besonders bevorzugt sind 1-Propanol und 2-Butanol. Hydroxylamin wird vorteilhaft in Form einer wässrigen Lösung eingesetzt, entweder in freier Form oder in Form eines Salzes, wie z.B. als Hydrochlorid, als Sulfat oder als Phosphat.

Besonders vorteilhaft ist es, für diese Reaktion das gleiche Lösungsmittel zu verwenden wie für die Folgestufe und somit die Isolierung der Zwischenstufe zu vermeiden.

### A) Herstellungsbeispiele für 7-Chlorbenzisothiazol (Verbindung IA)

### Beispiel 1:

243,7 g (1 Mol) 3-Chlor-2-(tert.butylthio)benzaldehydoxim und 9.5 g (0.05 Mol) p-Toluolsulfonsäure-monohydrat werden in 700 ml 1-Propanol während 2-4 Stunden am Rückfluss (95-100°C) gehalten; während dieser Zeit wird laufend Lösungsmittel abdestilliert. Danach wird das zurückbleibende Reaktionsgemisch mit Wasser versetzt und das auskristallisierte Produkt abfiltriert und getrocknet; Ausbeute 165 g (97% d.Th.); Gehalt >95%, Smp. 49°C.

### Beispiel 1A:

### Vergleichsbeispiel zur Herstellung von 7-Chlorbenzisothiazol gemäss Stand der Technik

243,7 g (1 Mol) 3-Chlor-2-(tert.butylthio)benzaldehydoxim werden in 700 ml Polyphosphorsäure während 1.5 Stunden bei 65-70°C gehalten; nach dieser Zeit ist gemäss Dünnschichtchromatographie alles Edukt umgesetzt. Danach wird bei 20-30°C Wasser zugegeben und das auskristallisierte Produkt abfiltriert und getrocknet. Ausbeute 75 g (44% d.Th.).

### Beispiel 2:

243,7 g (1 Mol) 3-Chlor-2-(n-butylthio)benzaldehydoxim und 19 g (0.1 Mol) p-Toluolsulfonsäure-monohydrat werden in 700 ml 1-Propanol während 2-4 Stunden am Rückfluss (95-100°C) gehalten; während dieser Zeit wird laufend Lösungsmittel abdestilliert. Danach wird das zurückbleibende Reaktionsgemisch mit Wasser und Toluol versetzt, die Toluolphase abgetrennt und das Toluol abdestilliert. Ausbeute 197 g (66% d.Th.); Gehalt 57%.

### Beispiel 3:

Es wird wie in Beispiel 2 vorgegangen; aber anstelle von 3-Chlor-2-(n-butylthio)benzaldehydoxim wird 3-Chlor-2-(isopropylthio)benzaldehydoxim eingesetzt. Ausbeute 171 g (64% d.Th.); Gehalt 64%.

### Beispiel 4:

243,7 g (1 Mol) 3-Chlor-2-(tert.butylthio)benzaldhydoxim werden als Schmelze bei 110-115°C auf 200 g "Amberlyst 15" (stark saurer Kationenaustauscher) dosiert. Dabei entwickelt sich Isobutylen. Nach beendeter Zusosierung wird der Katalysator von der heissen Schmelze abfiltriert; das ölige Filtrat kristallisiert beim Abkühlen auf Raumtemperatur aus. Ausbeute 161 g (77% d.Th.); Gehalt 82%.

### B) Herstellungsbeispiele für Edukte

### Beispiel 5: Herstellung von 3-Chlor-2-(tert.butylthio)benzaldehydoxim (Verbindung IIA)

Zu einer Lösung von 228.7 g (1 Mol) 3-Chlor-2-(tert.butylthio)benzaldehyd in 500 ml 1-Propanol werden 35 g (1.05 Mol) Hydroxylamin in Form einer 50% wässrigen Lösung innert 1-2 Stunden bei 60°C zudosiert. Am Ende der Zudosierung ist alles Edukt umgesetzt.
Diese Lösung kann tel quel für die Folgestufe verwendet werden.
Zur Isolierung des Produktes werden ca. 300 ml des Lösungsmittels abdestilliert, 500 ml Wasser zugegeben und danach nochmals ca. 200 ml abdestilliert; das Produkt kristallisiert aus und wird abfiltriert. Ausbeute: 241 g (99% d.Th.); Smp. 119-121°C.

### Beispiel 6: Herstellung von 3-Chlor-2-(tert.butylthio)benzaldehyd (Verbindung IIIA)

Zu einer Suspension von 175 g (1 Mol) 2,3-Dichlorbenzaldehyd und 155 g (1.12 Mol) Kaliumcarbonat in 460 ml N,N-Dimethylformamid werden bei 110°C 98 g (1.1 Mol) tert.Butylmercaptan innert 4-5 Stunden zudosiert; danach wird noch 4 Stunden bei 120°C gerührt. Anschliessend wird das Lösungsmittel im Vakuum abdestilliert, der Rückstand bei 70°C mit 300 ml Wasser ausgewaschen, die organische Phase bei 70°C mit 200 ml Wasser versetzt und unter Rühren auf 30-40°C abgekühlt. Dabei kristallisiert das Produkt aus und wird abfiltriert. Ausbeute: 195 g (85% d.Th.); Gehalt >96%: Smp. 52-54°C.

## Patentansprüche

1. Verfahren zur Herstellung einer Verbindung der Formel I, 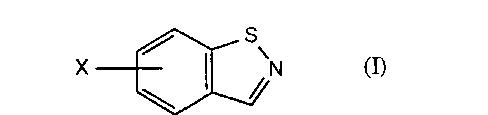 worin
X in einer der 4 möglichen Positionen des Benzolringes steht und Wasserstoff, Halogen, Nitro, C₁-C₄-Alkoxy, Halogen-C₁-C₄-alkoxy, C₂-C₄-Alkenyloxy oder C₂-C₄-Alkinyloxy bedeuten,
dadurch gekennzeichnet, dass eine Verbindung der Formel II 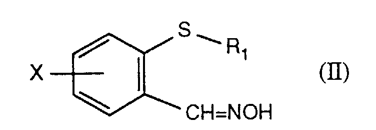 worin X die für Formel I angegebenen Bedeutungen hat und
R₁ Wasserstoff oder einen gegebenenfalls substituierten C₁-C₁₂-Kohlenwasserstoffrest bedeutet, in Gegenwart einer katalytischen Menge einer starken Säure von 1-50 Mol% bezogen auf die Verbindung der Formel II umgesetzt wird.

2. Verfahren gemäss Anspruch 1, dadurch gekennzeichnet, dass
R₁ einen gegebenenfalls substituierten C₁-C₁₂-Kohlenwasserstoffrest bedeutet und dass die Reaktion in einem Lösungsmittel durchgeführt wird.

3. Verfahren gemäss Anspruch 1, worin X Halogen oder Nitro bedeuten.

4. Verfahren gemäss Anspruch 3, worin X in 7-Stellung steht.

5. Verfahren gemäss Anspruch 4, worin X Chlor bedeutet.

6. Verfahren gemäss Anspruch 1, worin in Formel II
R₁ C₁-C₆-Alkyl oder Benzyl bedeuten.

7. Verfahren gemäss Anspruch 6, worin R₁ tert.Butyl bedeutet.

8. Verfahren gemäss Anspruch 1, worin die Reaktion in einem polaren Lösungsmittel durchgeführt wird.

9. Verfahren gemäss Anspruch 8, worin als Lösungsmittel Wasser oder ein C₁-C₆-Alkohol oder ein Gemisch derselben verwendet wird.

10. Verfahren gemäss Anspruch 9, worin als Lösungsmittel 1-Propanol oder 2-Butanol verwendet wird.

11. Verfahren gemäss Anspruch 1, worin als Katalysator eine Mineralsäure oder eine Sulfonsäure eingesetzt wird.

12. Verfahren gemäss Anspruch 11, worin als Katalysator p-Toluolsulfonsäure, Methansulfonsäure, Hydroxylamin-O-sulfonsäure oder Schwefelsäure eingesetzt wird.

13. Verfahren gemäss Anspruch 1, worin als Katalysator eine an ein festes Trägermaterial gebundene Säure eingesetzt wird.

14. Verfahren gemäss Anspruch 1, worin der Katalysator in einer Menge von 2-15 Mol% bezogen auf die Verbindung der Formel II eingesetzt wird.

15. Verfahren gemäss Anspruch 1, dadurch gekennzeichnet, dass die Reaktion bei 30°C bis zur Rückflusstemperatur des Lösungsmittels durchgeführt wird.

16. Verfahren gemäss Anspruch 15, dadurch gekennzeichnet, dass die Reaktion bei Rückflusstemperatur des Lösungsmittels durchgeführt und laufend Lösungsmittel abdestilliert wird.

17. Verfahren gemäss Anspruch 1, dadurch gekennzeichnet, dass eine Verbindung der Formel II kontinuierlich über eine an ein festes Trägermaterial gebundene Säure geleitet wird.

18. Verfahren zur Herstellung einer Verbindung der Formel I gemäss Anspruch 1, dadurch gekennzeichnet, dass eine Verbindung der Formel III, 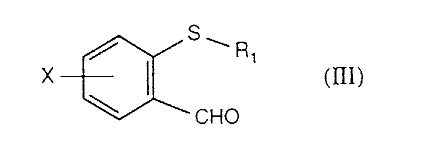 worin R₁ und X die für Formel II in Anspruch 1 angegebenen Bedeutungen haben, mit Hydroxylamin in freier Form oder in Salzform, in einem polaren Lösungsmittel zu einer Verbindung der Formel II 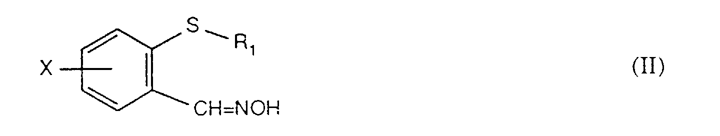 und diese Verbindung ohne Isolierung gemäss Anspruch 1 weiter umgesetzt wird.

19. Verfahren gemäss Anspruch 18, dadurch gekennzeichnet, dass als Lösungsmittel 1-Propanol oder 2-Butanol verwendet wird.

## Claims

1. A process for the preparation of a compound of formula I 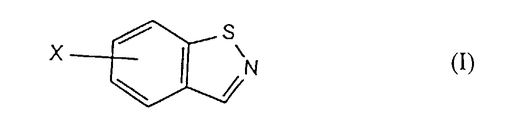 wherein
X is in any one of the 4 possible positions of the benzene ring and is hydrogen, halogen, nitro, C₁-C₄alkoxy, halo-C₁-C₄alkoxy, C₂-C₄alkenyloxy or C₂-C₄alkynyloxy,
in which process a compound of formula II 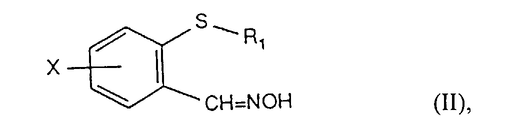 wherein X is as defined for formula I and
R₁ is hydrogen or an unsubstituted or substituted C₁-C₁₂hydrocarbon radical,
is reacted in the presence of a catalytic amount of a strong acid of from 1 to 50 mol% based on the compound of formula II.

2. A process according to claim 1, wherein R₁ is an unsubstituted or substituted C₁-C₁₂hydrocarbon radical and the reaction is carried out in a solvent.

3. A process according to claim 1, wherein X is halogen or nitro.

4. A process according to claim 3, wherein X is in the 7-position.

5. A process according to claim 4, wherein X is chlorine.

6. A process according to claim 1, wherein in formula II R₁ is C₁-C₆alkyl or benzyl.

7. A process according to claim 6, wherein R₁ is tert-butyl.

8. A process according to claim 1, wherein the reaction is carried out in a polar solvent.

9. A process according to claim 8, wherein water or a C₁-C₆alcohol or a mixture thereof is used as the solvent.

10. A process according to claim 9, wherein 1 -propanol or 2-butanol is used as the solvent.

11. A process according to claim 1, wherein a mineral acid or a sulfonic acid is used as the catalyst.

12. A process according to claim 11, wherein p-toluenesulfonic acid, methanesulfonic acid, hydroxylamine-O-sulfonic acid or sulfuric acid is used as the catalyst.

13. A process according to claim 1, wherein an acid bound to a solid carrier is used as the catalyst.

14. A process according to claim 1, wherein the catalyst is used in an amount of from 2 to 15 mol% based on the compound of formula II.

15. A process according to claim 1, wherein the reaction is carried out at from 30°C up to the reflux temperature of the solvent.

16. A process according to claim 15, wherein the reaction is carried out at the reflux temperature of the solvent and solvent is distilled off continuously.

17. A process according to claim 1, wherein a compound of formula II is conveyed continuously over an acid bound to a solid carrier.

18. A process for the preparation of a compound of formula I according to claim 1, wherein a compound of formula III 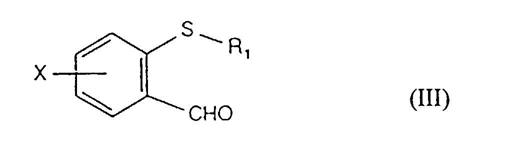 wherein R₁ and X are as defined for formula II in claim 1 is reacted with hydroxylamine in free form or in salt form in a polar solvent to form a compound of formula II 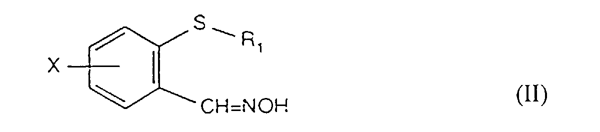 and that compound is reacted further, without being isolated, according to claim 1.

19. A process according to claim 18, wherein 1 -propanol or 2-butanol is used as the solvent.

## Revendications

1. Procédé pour la préparation d'un composé de formule I 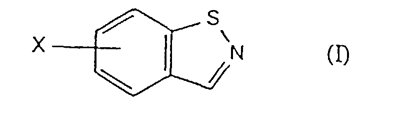 dans laquelle
X se trouve en l'une des 4 positions possibles du cycle benzénique et représente un atome d'hydrogène ou d'halogène ou un groupe nitro, alcoxy en C₁-C₄, halogénoalcoxy en C₁-C₄, alcényloxy en C₂-C₄ ou alcynyloxy en C₂-C₄,
caractérisé en ce que l'on fait réagir un composé de formule II 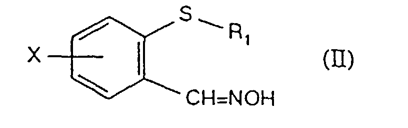 dans laquelle X a les significations indiquées pour la formule I et R₁ représente un atome d'hydrogène ou un radical hydrocarboné en C₁-C₁₂ éventuellement substitué, en présence d'une quantité catalytique d'un acide fort, représentant de 1 à 50 % en moles, par rapport au composé de formule II.

2. Procédé selon la revendication 1, caractérisé en ce que
R₁ représente un radical hydrocarboné en C₁-C₁₂ éventuellement substitué et en ce que la réaction est effectuée dans un solvant.

3. Procédé selon la revendication 1, dans lequel X représente un atome d'halogène ou le groupe nitro.

4. Procédé selon la revendication 3, dans lequel X est en position 7.

5. Procédé selon la revendication 4, dans lequel X représente un atome de chlore.

6. Procédé selon la revendication 1, dans lequel, dans la formule II,
R₁ représente un groupe alkyle en C₁-C₆ ou benzyle.

7. Procédé selon la revendication 6, dans lequel R₁ représente le groupe tert-butyle.

8. Procédé selon la revendication 1, dans lequel la réaction est effectuée dans un solvant polaire.

9. Procédé selon la revendication 8, dans lequel on utilise comme solvant l'eau ou un alcool en C₁-C₆ ou un mélange de ceux-ci.

10. Procédé selon la revendication 9, dans lequel on utilise comme solvant le 1-propanol ou le 2-propanol.

11. Procédé selon la revendication 1, dans lequel on utilise comme catalyseur un acide minéral ou un acide sulfonique.

12. Procédé selon la revendication 11, dans lequel on utilise comme catalyseur l'acide p-toluènesulfonique, méthanesulfonique, hydroxylamine-O-sulfonique ou sulfurique.

13. Procédé selon la revendication 1, dans lequel on utilise comme catalyseur un acide fixé à un matériau de support solide.

14. Procédé selon la revendication 1, dans lequel le catalyseur est utilisé en une proportion de 2-15 % en moles, par rapport au composé de formule II.

15. Procédé selon la revendication 1, caractérisé en ce que la réaction est effectuée à une température dans la plage allant de 30°C à la température de reflux du solvant.

16. Procédé selon la revendication 15, caractérisé en ce que la réaction est effectuée à la température de reflux du solvant et le solvant est éliminé en continu par distillation.

17. Procédé selon la revendication 1, caractérisé en ce que l'on fait passer en continu un composé de formule II sur un acide fixé à un matériau de support solide.

18. Procédé pour la préparation d'un composé de formule I selon la revendication 1, caractérisé en ce que l'on fait réagir un composé de formule III 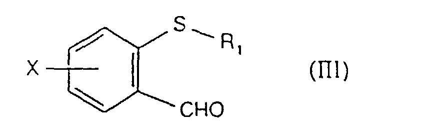 dans laquelle R₁ et X ont les significations données pour la formule II dans la revendication 1, avec de l'hydroxylamine sous forme libre ou sous forme de sel, dans un solvant polaire, pour aboutir à un composé de formule Il 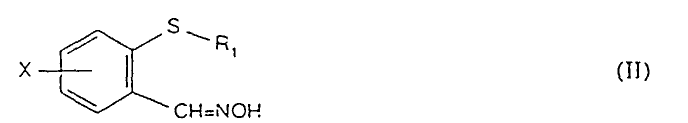 et on soumet ce composé sans l'isoler à la réaction suivante selon la revendication 1.

19. Procédé selon la revendication 18, caractérisé en ce que l'on utilise comme solvant le 1-propanol ou le 2-butanol.
